# EUROPEAN PATENT APPLICATION

(11) **EP 3 811 932 A1**
(43) Date of publication of application: **28.04.2021**
(21) Application number: 19204725.6
(22) Date of filing: 22.10.2019
(51) Int. Cl.: A61K 9/16, A61K 9/20, A61K 31/4439

(54) **DOSAGE FORM OF APALUTAMIDE**

(71) Applicant: Zentiva K.S., 102 37 Praha 10 (CZ)
(72) Inventor: Cernova, Miroslava, 16900 Praha 6 (CZ); Smrcka, David, 75701 Valasske Mezirici (CZ); Man, Stanislav, 68732 Nezdenice (CZ); Krejcik, Lukas, 19017 Praha 9 - Vinor (CZ); Simek, Michal, 14000 Praha 4 (CZ)
(74) Representative: Hartvichova, Katerina

(57) **Abstract**

The present invention provides a solid dosage form of apalutamide which comprises a solid dispersion of apalutamide comprising or consisting of apalutamide and hydroxypropyl methylcellulose phthalate, preferably in weight ratio about 1:3.

Such solid dosage form has an improved dissolution profile and allows to achieve a higher drug loading and a smaller size of dosage form.

## Description

### Field of Art

The present invention relates to a pharmaceutical formulation of solid dispersion of apalutamide.

### Background Art

Apalutamide (chemical name: 4-[7-[6-cyano-5-(trifluoromethyl)pyridin-3-yl]-8-oxo-6-thioxo-5,7-diazaspiro[3.4]octan-5-yl]-2-fluoro-N-methylbenzamide) is an androgen receptor antagonist used in the treatment of metastatic castration-resistant prostate cancer. Apalutamide is a white to slightly yellow powder with the molecular weight 477.44 g/mol and molecular formula C₂₁H₁₅F₄N₅O₂S. The structural formula is:

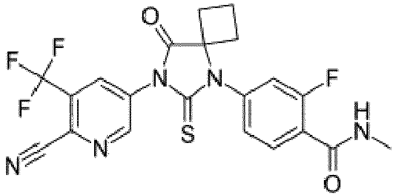

Apalutamide is poorly soluble, practically insoluble in aqueous media over a wide range of pH values. Further, it is non-hygroscopic, crystalline solid that remains unionized over the physiologic pH range. It belong to Class 2 drugs using the Biopharmaceutics Classification System. Poor drug solubility however represents a bottleneck for dissolution, which in turn critically affects drug bioavailability.

WO 2015/118015 was the first document to provide a fully solid dosage form of apalutamide which comprises apalutamide, a porous carrier and a surfactant. The surfactant and porous carrier ensure that the composition can be fully solid, with good solubility. The dosage form may further comprise a hydrophilic polymer functioning as a wettability enhancer. Preferred polymers are HPMC, HPMC AS, HPC, PVP and PVA.

WO 2016/090098 and WO 2016/060105 disclose a mixture comprising apalutamide and HPMCAS, or apalutamide and HPMCAS and poly(meth)acrylate copolymer, respectively. The formulations are claimed to have an improved shelf life and an improved solubility. The original drug product (ODP) Erleada which is currently on the market uses HPMCAS.

WO 2019/016747 discloses solid dispersions of apalutamide with various polymers, including hydroxypropyl methylcellulose phthalate, copovidone, cellulose acetate phthalate, polyvinyl acetate phthalate, ethyl cellulose, hydroxypropyl cellulose, methyl cellulose, prosolv, hydroxypropyl methylcellulose or mixtures thereof. The solid dispersions are prepared at varying ratios of the active ingredient to polymer. They are mostly dried using removal of the solvent at a decreased pressure. The aim of the solid dispersion forms of apalutamide is to provide stable forms of apalutamide with a reproducible preparation process. However, this document does not relate to solid dosage forms.

The present invention aims at providing a solid dosage form with an improved dissolution profile in relation to the original drug product, and allowing to achieve a higher drug loading and/or smaller size of dosage forms.

### Disclosure of the Invention

The present invention provides a solid dosage form of apalutamide which comprises a solid dispersion of apalutamide comprising or consisting of apalutamide and hydroxypropyl methylcellulose phthalate (HPMCP).

HPMCP or hypromellose phthalate dissolves at pH 5-5.5 and this property can be controlled by varying the phthalyl content. Two grades of different pH solubility, HP-55 and HP-50, are available from Shin-Etsu Chemical Co., Ltd. In some embodiments of the present invention, hydroxypropyl methylcellulose phthalate grade HP-55 is preferred.

Within the framework of the present invention, it was found that HPMCP allows to achieve a higher concentration of apalutamide before the precipitation occurs, compared to other pharmaceutically acceptable polymers. This allows to achieve a higher drug loading due to lower needed content of the polymer, and results in smaller total size of dosage forms which increases convenience for the patient and improves patient compliance. Furthermore, the faster dissolution profile achievable in HPMCP-containing formulations results in a better bioavailability and synergizes with the supersaturation effect of HPMCP.

In an aspect of the invention, the weight ratio of apalutamide:HPMCP in the solid dispersion is 1:3.

In an aspect of the invention, the solid dispersion of apalutamide and hydroxypropyl methylcellulose phthalate is obtainable by spray drying a mixture comprising apalutamide and HPMCP in a solvent.

In a preferred aspect of the invention, the dosage form comprises 60 mg of apalutamide.

In an aspect of the invention, the solid dosage form is a tablet, a coated tablet, or a capsule.

In an aspect of the invention, the solid dispersion is a solid solution. A solid solution typically results from spray drying. In the following text, the term "solid dispersion" should be understood as including also solid solution.

Preferred is a solid dispersion, more preferably a solid solution, of apalutamide and HPMCP wherein substantially all apalutamide is in an amorphous (non-crystalline) phase. Such solid dispersion has a faster dissolution rate than a dispersion in which part or all of apalutamide is in a crystalline form.

In an aspect of the invention, the solid dispersion is in the form of an amorphous solid solution.

The term "solid dispersion" means a system in a solid state comprising at least two components, wherein one component is dispersed (more or less evenly) throughout the other component or components. When said dispersion of the components is such that the system is chemically and physically uniform or homogenous throughout or consists of one phase as defined in thermodynamics, such a solid dispersion will be called "solid solution" herein. Solid solutions are preferred physical systems because the components therein are usually more readily bioavailable to the organisms to which they are administered. This advantage can probably be explained by the ease with which said solid solutions can form liquid solutions when contacted with a liquid medium such as gastric juice. The ease of dissolution may be attributed at least in part to the fact that the energy required for dissolution of the components from a solid solution is less than that required for the dissolution of components from a crystalline or microcrystalline solid phase.

The term "amorphous" as applied to a compound or a mixture of compounds refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterized by a change of state, typically second order ("glass transition").

The solid dispersions are formulated into solid dosage forms comprising a therapeutically effective amount of apalutamide (such as 60 mg). A preferred solid dosage form is a tablet which can be produced by conventional tableting techniques and with conventional tableting machines. A film coat on the tablet may further contribute to the ease with which the tablet can be swallowed.

Preferred dosage forms are those wherein the weight of solid dispersion as described herein ranges from 20 to 40 %, in particular from 30 to 40 % of the total weight of the formulation.

The solid dosage form of the invention may include one or more pharmaceutically acceptable excipients such as disintegrants, diluents, fillers, binders, lubricants, glidants, thickening agents, sweetening agents, flavors, and/or colorants. Some excipients can serve multiple purposes. Preferably, the solid dosage form of the present invention includes at least one disintegrant, at least one filler, at least one lubricant and at least one glidant.

Suitable disintegrants are those that have a large coefficient of expansion. Examples thereof are hydrophilic, insoluble or poorly water-soluble crosslinked polymers such as crospovidone (crosslinked polyvinylpyrrolidone) and croscarmellose sodium (crosslinked sodium carboxymethylcellulose). Preferred is croscarmellose sodium. The amount of disintegrant in the solid dosage form may conveniently range from about 3 to about 15 % (w/w) and preferably range from about 3 to about 7 % (w/w).

Suitable diluents or fillers include lactose monohydrate, anhydrous lactose, sucrose, dextrose, mannitol, sorbitol, starch, cellulose (e.g. micro-crystalline cellulose (Avicel™), silicified microcrystalline cellulose), dihydrated or anhydrous dibasic calcium phosphate, and mixtures thereof. Preferred is microcrystalline cellulose and silicified microcrystalline cellulose. The amount of diluent or filler in the tablets may conveniently range from about 20 % to about 70 % (w/w) and preferably ranges from about 55 % to about 60 % (w/w).

Suitable lubricants and glidants include magnesium stearate, stearic acid, sodium stearyl fumarate, sodium lauryl sulfate, magnesium lauryl sulfate, colloidal silica, colloidal anhydrous silica, talc, mixtures thereof. Preferred lubricants are magnesium stearate, and mixtures of magnesium stearate with colloidal silica. An especially preferred lubricant is magnesium stearate. A preferred glidant is colloidal anhydrous silica. Glidants generally represent 0.2 to 7.0 % of the total tablet weight, in particular 0.5 to 1.5%, more particularly 1 to 1.5% (w/w). Lubricants generally represent 0.2 to 7.0 % of the total tablet weight, in particular 0.2 to 1%, more particularly 0.5 to 1% (w/w).

In some embodiments, the solid dosage form of the present invention contains:
- a solid dispersion of apalutamide comprising or consisting of apalutamide and hydroxypropyl methylcellulose phthalate, preferably in the weight ratio about 1:3;
- a disintegrant which is croscarmellose sodium;
- at least one filler selected from microcrystalline cellulose, silicified microcrystalline cellulose and mixture thereof;
- a lubricant which is magnesium stearate; and
- a glidant which is colloidal anhydrous silica.

In some embodiments, the solid dosage form of the present invention contains
- 20-40 % (w/w), preferably 30-40 % (w/w), of solid dispersion of apalutamide comprising or consisting of apalutamide and hydroxypropyl methylcellulose phthalate in the weight ratio about 1:3;
- 3-15 % (w/w), preferably 3-7 % (w/w), of disintegrant which is croscarmellose sodium;
- 20-70 % (w/w), preferably 55-60 % (w/w), of filler selected from microcrystalline cellulose, silicified microcrystalline cellulose and mixture thereof;
- 0.2-7 % (w/w), preferably 0.2-1 % (w/w), of lubricant which is magnesium stearate; and
- 0.2-7 % (w/w), preferably 0.5-1.5 % (w/w), of glidant which is colloidal anhydrous silica.

The present invention further provides a method for preparing the solid dosage form of the present invention, said method comprising the following steps:
a) providing a solution of apalutamide and HPMCP in a solvent,
b) isolating a solid dispersion of apalutamide and HPMCP,
c) combining the solid dispersion with at least one pharmaceutically excipient,
d) producing the solid dosage form.

Solvents suitable for use in step a) can be any organic solvent with which apalutamide and HPMCP are miscible. In an aspect of the invention, the boiling point of the solvent is lower than the Tg (glass transition temperature) of the solid dispersion. In addition, the solvent should have a relatively low toxicity and be removable from the dispersion to a level that is acceptable according to The International Committee on Harmonization (ICH) guidelines. Removal of the solvent to this level may require a post drying step such as for instance a drying in a vacuum oven, subsequent to the isolation of the solid dispersion in step b). Solvents can include alcohols such as methanol, ethanol, n-propanol, iso-propanol, and butanol, in particular methanol; ketones such as acetone, methyl ethyl ketone and methyl iso-butyl ketone; esters such as ethyl acetate and propylacetate; halogenated C1-C3 alkanes such as dichloromethane, 1,1,1-trichloroethane; and various other solvents such as acetonitrile or toluene. Lower volatility solvents such as dimethyl acetamide or dimethylsulfoxide can also be used. In an aspect of the invention, the solvent is a mixture of at least two solvents. Preferably, the solvent in step a) is a mixture of an alcohol and dichloromethane, in particular a mixture of methanol and dichloromethane, more preferably a mixture of methanol and dichloromethane 6:4 (w:w).

In principle, any crystalline, semi-crystalline or non-crystalline form of apalutamide may be useful for preparing the solid dispersion. A preferred crystalline form of apalutamide for preparing the solid dispersion as described herein is Form A (an ethanol solvate), Form B (an unsolvated crystalline form) or Form C (an isopropanol solvate).

A particularly preferred method of isolating the solid dispersion is a spray-drying process. The spray-drying process typically comprises the following steps:
b1) injecting the solution of apalutamide and HPMCP obtained in step a) to a spray dryer,
b2) atomization of the solution to create droplets,
b3) drying the droplets in an air stream, said air stream preferably having temperature within the range of 50 to 200 °C,
b4) collecting the dried particles.

In step c), the obtained solid dispersion is combined with at least one pharmaceutically acceptable excipient by conventional procedures such as stirring, mixing, direct compression or granulation (dry-granulation or wet granulation) or a combination of such procedures.

In a preferred embodiment of step c), the solid dispersion is first blended with a part (50 to 80 %) of the glidant, this blend is further blended with a part (25 to 75 %) of the disintegrant and with the a part (50 to 80 %) of the filler, the resulting mixture is subjected to dry granulation, and the dry granulate is then blended with the remaining amounts of the filler, glidant and disintegrant, and finally the mixture is blended with the lubricant.

In step d), the solid dosage form may be produced by conventional procedures. For example, tablet blends may be directly compressed or may be dry-granulated or wet-granulated before tableting. The tableting process itself is otherwise standard and readily practiced by forming a tablet from the blend or mixture of ingredients into the appropriate shape using a conventional tablet press. The tablets may then be coated. For example, the capsules may be produced by filling of empty capsules with powder or pellets of the blend of solid dispersion with the pharmaceutically acceptable excipient(s).

The present invention further provides the solid dosage form as described herein for use in the treatment of prostate cancer, more specifically in the treatment of metastatic castration-resistant prostate cancer.

### Brief Description of Drawings

Figure 1: Precipitation test (Example 1). Comparison of supersaturation concentration of apalutamide in the buffer with addition of HPMCAS and HPMCP.
Figure 2: Precipitation test (Example 2). Comparison of supersaturation concentration of apalutamide in the buffer with addition of the following polymer: a) Copovidone VA64 b) Klucel EF, c) Methocel K100, d) HPMCAS and e) HPMCP.
Figure 3: XRPD difractogram of solid solution prepared in Example 3.
Figure 4: XRPD difractogram of tablet formulation prepared in Example 4.
Figure 5: DSC curve of amorphous solid solution prepared in Example 3.
Figure 6: DSC curve of tablet formulation (amorphous solid solution) prepared in Example 4.
Figure 7: Dissolution behavior of the tablet composition containing solid solution of apalutamide with polymer HPMCP, prepared according to Example 4, compared with the ODP ERLEADA.
Figure 8: Solid solutions of apalutamide with polymers (HPMCAS, HPMCP and Klucel EF) formulated into the tablets were subjected to dissolution test, where pH was switched from acidic pH 2 into neutral pH 6.8, simulating fasting conditions in gastro-intestinal conditions (Example 6).

### Examples of carrying out the Invention

The following examples are intended to illustrate the present invention, without limiting the scope thereof.

### Example 1: Polymer performance testing with respect to preventing precipitation of apalutamide

The aim of this test is to investigate the level of apalutamide supersaturation in phosphate buffer pH 6.8 containing dissolved polymer with given concentration (90 mg/L). Polymer performance was evaluated by the ability to maintain the supersaturation and respective concentration of dissolved apalutamide. The HPMCAS served as a reference polymer used in the original drug product (Erleada) with ability to maintain 77 % of apalutamide dissolved. The HPMCP exhibited ability to maintain a higher dissolved concentration of apalutamide (approximately 87 %). From thermodynamical point of view, the HPMCP has potential to be formulated in the solid solution and perform comparably as original drug product even with a lowered load of the polymer, which in turn implies smaller tablets which are more convenient for the patient.

### Method:

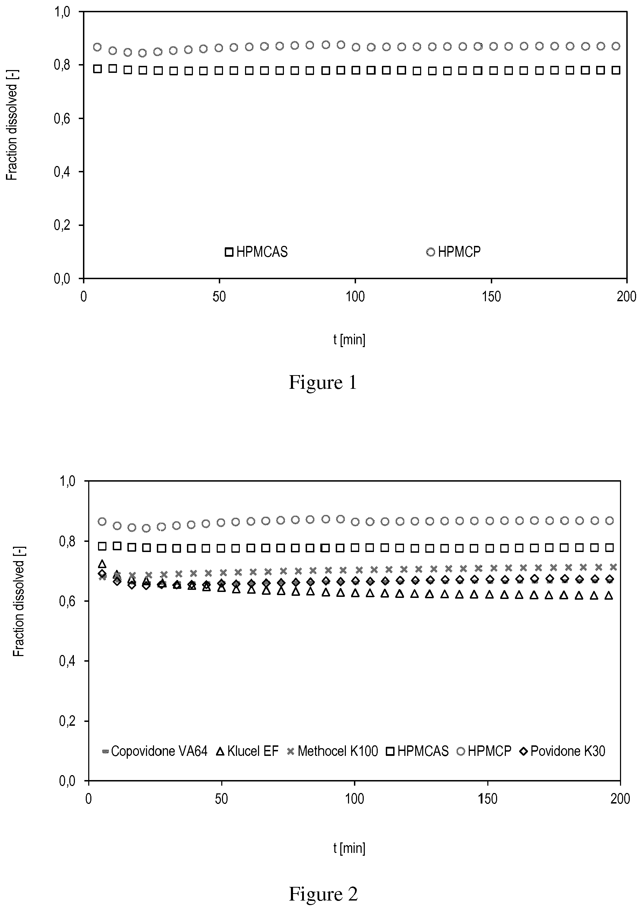

4.5 mg of investigated polymer (HPMCAS or HPMCP) was dissolved in 50 ml of 72.4mM phosphate buffer pH 6.8. This solution was used as a medium for a precipitation test, where 250 µL of 30mM apalutamide solution in DMSO was injected into the phosphate buffer mixed by a paddle stirrer (100 rpm). Concentration of dissolved apalutamide was online monitored via UV spectrophotometry. Precipitation tests were performed on a microscale dissolution apparatus inForm (Pion, UK). The results are presented in Figure 1.

### Example 2: Polymer performance testing with respect to preventing precipitation of apalutamide

The polymers Copovidone VA64, Klucel EF, Methocel K100, Povidone K30, HPMCAS and HPMCP were tested in precipitation tests. The polymers Copovidone VA64, Klucel EF, Methocel K100, Povidone K30 maintained lower concentration in the range from 62% to 71%, which was worse than the HPMCAS reference (Figure 2). The polymer HPMCP showed the best effect of all tested polymers, and performed better than the HPMCAS reference.

### Method:

4.5 mg of the tested polymer (HPMCAS, HPMCP, Copovidone VA64, Klucel EF, Methocel K100 and Povidone K30, respectively) was dissolved in 50 ml of 72.4 mM phosphate buffer pH 6.8. This solution was used as a medium for a precipitation test, where 250 µL of 30 mM apalutamide solution in DMSO was injected into the phosphate buffer mixed by a paddle stirrer (100 rpm). Concentration of dissolved apalutamide was online monitored via UV spectrophotometry. Precipitation tests were performed on a microscale dissolution apparatus inForm (Pion, UK).

### Example 3: Preparation of a solid dispersion of apalutamide:HPMCP 1:3

| **Ingredient** | **Amount** [g] |
|---|---|
| Apalutamide | 2.5 |
| HPMCP HP-55 | 7.5 |
| Dichloromethane | 76.0 (58 ml) |
| Methanol | 114.0 (128 ml) |

Dichloromethane and methanol were transferred into a suitable container and stirring was started. Under continuous stirring apalutamide was added to the solvent mixture and stirred until dissolved. HPMCP was added to the solution and stirred overnight. A viscous mixture was obtained. The mixture was spray dried using Büchi Mini Spray Dryer B-290 with a high pressure nozzle with the following parameters: feed flow of 700 ml/hour, inlet temperature of 100 °C, outlet temperature of 45-50 °C and a condenser temperature of -20 °C. The spray dried product (SDP) was dried in a vacuum oven, drying temperature of 40 °C, within one day. The spray dried solid dispersion corresponded in physicochemical parameters to a solid solution.

### Example 4: Preparation of tablets comprising a solid dispersion of apalutamide:HPMCP 1:3

| **Ingredient** | **Amount in tablet** | |
|---|---|---|
| | [mg] | [%] |
| Spray dried powder from Example 1 | 240.0 | 34.3 |
| Colloidal Anhydrous Silica | 9.1 | 1.3 |
| Croscarmellose sodium | 35.0 | 5.0 |
| Microcrystalline Cellulose | 295.5 | 42.2 |
| Silicified Microcrystalline Cellulose | 116.9 | 16.7 |
| Magnesium stearate | 3.5 | 0.5 |
| Total of tablet core | 700.0 | 100.0 |

The spray dried powder of Example 3 was sieved through a 0.6 mm sieve and mixed with part (10/13) of the colloidal anhydrous silica to a homogeneous blend using the Turbula shaker mixer (49 rpm) within 3 min. Part (1/2) of the croscarmellose sodium and the microcrystallline cellulose (all) were sieved through a 0.6 mm sieve and added to the blend and mixed using the Turbula shaker mixer (49 rpm) within 5 min. A dry granulate was made by using a briquetting on an IR tablet press using 13 mm punches. The dry granulate was obtained by subsequent sieving through a 0.8 mm sieve. The silicified microcrystalline cellulose, remainder of the croscarmellose sodium (1/2) and colloidal anhydrous silica (3/13) were sieved through 0.6 mm sieve and added to the dry granulate and mixed using the Turbula shaker mixer (49 rpm) within 5 min. The magnesium stearate was sieved through a 0.6 mm sieve, added to the blend and mixed using the Turbula shaker mixer (49 rpm) within 3 min. The blend was compressed into tablets (containing 60 mg of apalutamide) using a tablet press (IR tablet press with 13 mm punches), compression force 20 kN for 30s.

### Example 5: Characterization of a solid dispersion of apalutamide:HPMCP 1:3 and of the solid dosage form

### Powder XRD

Diffractograms were obtained with difractometer X*'*PERT PRO MPD PANalytical, spinner stage configuration, reflection mode, goniometer PW3050/60, continuous scan type, 2theta range 2-40°, step size 0.0167° 2-theta, time per step 300s.

Analysis condition: CuKα radiation (λ=1.542 Å), generator settings (45 kV/40 mA), primary optics setting: Soller slits 0.02 rad, automatic PDS, 10 mm mask, 1/4° anti-scatter slit, irradiated sample area 10 mm, secondary optics setting: 5.0 mm anti-scatter slit, Soller slits 0.02 rad, detector X*'*Celerator with maximal active length.

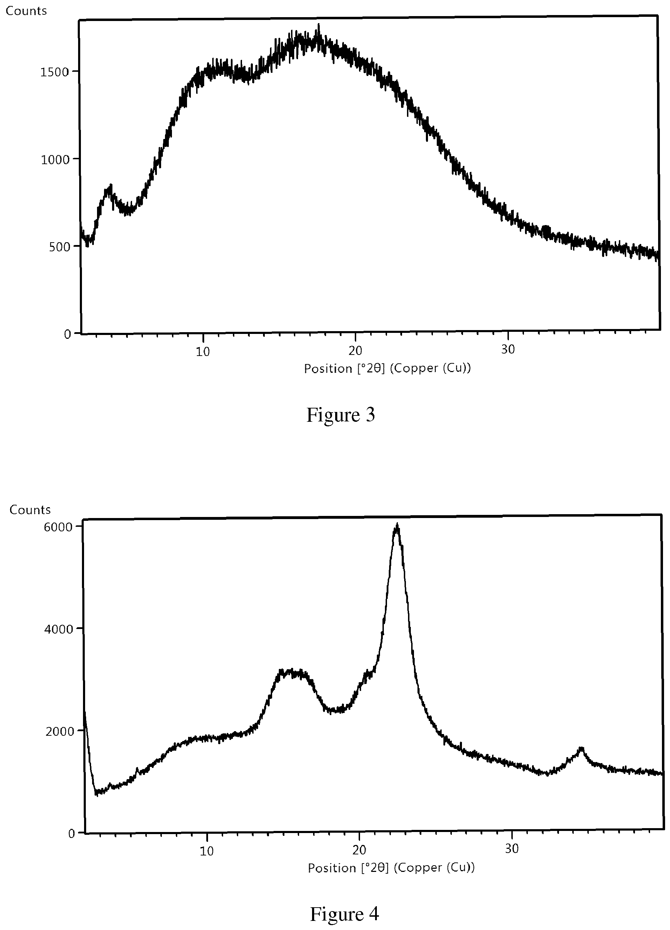

Figure 3 and Figure 4 are powder X-ray diffraction patterns corresponding to the amorphous solid solution and it's tablet formulation. Figure 3 shows that the solid solution is amorphous. Figure 4 shows that the tablet is mostly amorphous, with admixture of crystalline magnesium stearate with reflections at 3.66, 5.42 ± 0.2°2theta

### Differential scanning calorimetry (DSC)

### Modulation DSC

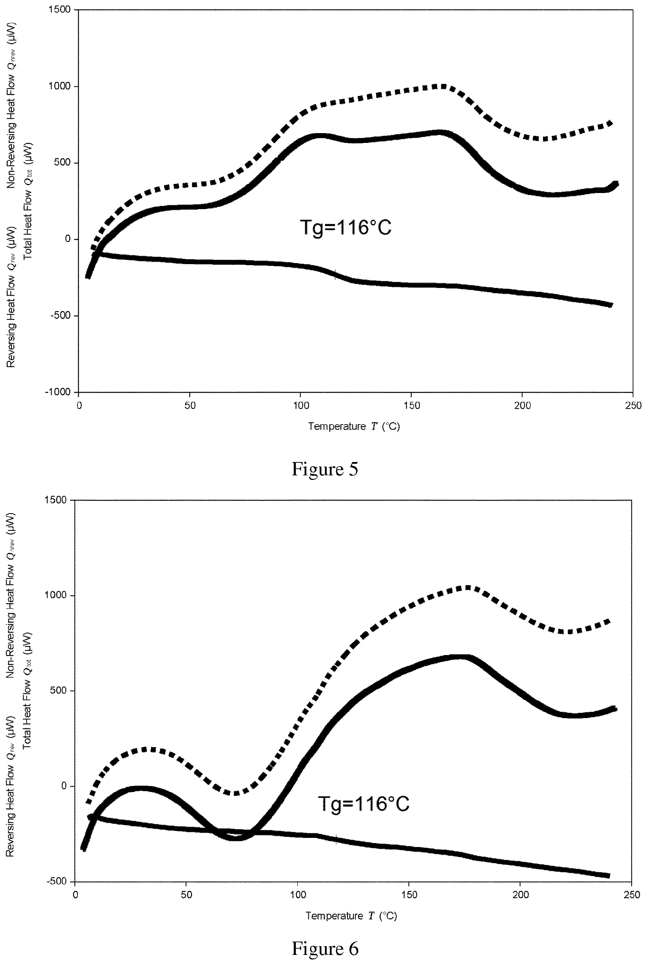

DSC measurements were performed on the TA Instruments Discovery DSC. The sample was weighed in an aluminum pan (40 µL), covered and measured in the flow of a nitrogen gas. The investigation was performed in the temperature range from 0°C to 250°C with the heating rate 5°C/min (Amplitude = 0.8°C; Period = 60s). The peak maximum temperature (Tₚₑₐₖ) and the peak onset temperature (Tₒₙₛₑₜ), for the crystalline form, and the glass transition temperature (Tg), for the amorphous form, were specified in the DSC result. The enthalpy was given by unit J/g. The weight sample was about 3-5 mg. Figure 5 and Figure 6 are DSC curves corresponding to the amorphous solid solution and its tablet formulation, both with a glass transition temperature Tg = 116 °C.

### Dissolution

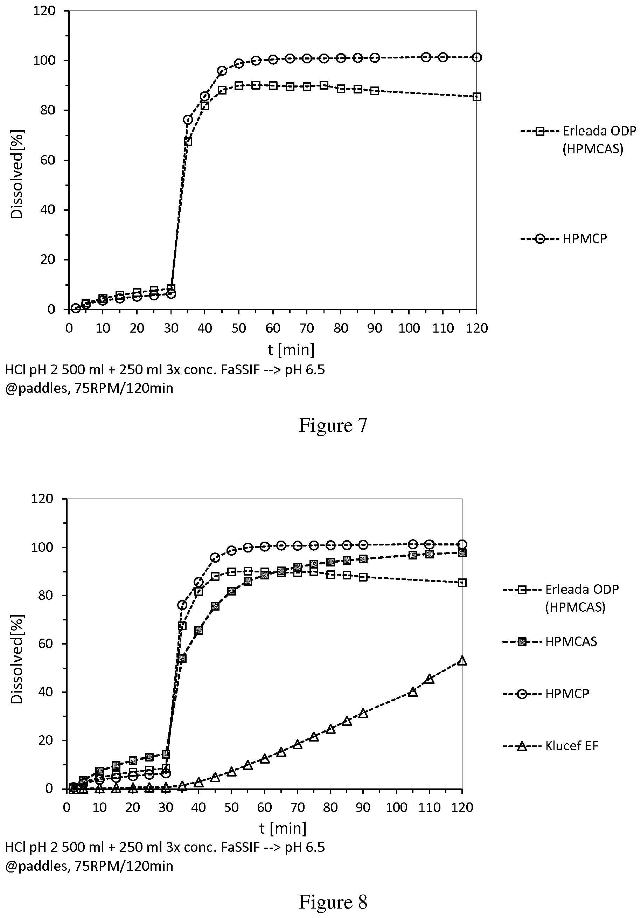

The tablets containing apalutamide in the form of solid solution with HPMCP were subjected to a dissolution test simulating the fasting conditions of gastrointestinal tract. During the first 30 minutes, the tablets were exposed to acidic pH 2, where the polymer did not dissolve and thus a release of apalutamide was limited. After 30 minutes the pH was adjusted to 6.5 by addition of 250 ml of 3 times concentrated FaSSIF. In neutral pH the polymer turned to be soluble and thus a rapid release of API was observed. HPMCP prototype exhibited fast release with comparable initial rate as original drug product ERLEADA (ODP) (Figure 7). According to the obtained dissolution profile, HPMCP exhibited a faster dissolution profile as HPMCAS reference. No precipitation was observed.

### Example 6 - Comparative example

The polymers HPMCAS, HPMCP and Klucel EF were used for preparation of the amorphous solid solutions by spray-drying (according to Example 31) and tablets were made of these solid solutions (according to Example 4). The tablets were subjected to a dissolution test simulating the fasting conditions of gastrointestinal tract. During the first 30 minutes, the tablets were exposed to acidic pH 2, where the polymer did not dissolve and thus a release of apalutamide was limited. After 30 minutes the pH was adjusted to 6.5 by addition of 250 ml of 3 times concentrated FaSSIF. In neutral pH the polymer turned to be soluble and thus a rapid release of API was observed. HPMCP prototype exhibited fast release with comparable initial rate as the original drug product ERLEADA (ODP). The prototype containing the solid solution with Klucel EF was the worst and the release of API was only 53% (Figure 8).

## Claims

1. A solid dosage form of apalutamide which comprises a solid dispersion of apalutamide comprising or consisting of apalutamide and hydroxypropyl methylcellulose phthalate.

2. The solid dosage form according to any one of the preceding claims, wherein the weight ratio of apalutamide:HPMCP in the solid dispersion is about 1:3.

3. The solid dosage form according to any one of the preceding claims, wherein the solid dispersion of apalutamide and hydroxypropyl methylcellulose phthalate is obtainable by spray drying a mixture comprising apalutamide and hydroxypropyl methylcellulose phthalate in a solvent.

4. The solid dosage form according to any one of the preceding claims, wherein the solid dispersion of apalutamide and hydroxypropyl methylcellulose phthalate is a solid solution of apalutamide and hydroxypropyl methylcellulose phthalate.

5. The solid dosage form according to any one of the preceding claims, wherein substantially all apalutamide is in an amorphous phase.

6. The solid dosage form according to any one of the preceding claims which further contains at least one of: disintegrant, filler, lubricant, glidant.

7. The solid dosage form according to any one of the preceding claims, which contains:
- a solid dispersion of apalutamide comprising or consisting of apalutamide and hydroxypropyl methylcellulose phthalate, preferably in the weight ratio about 1:3;
- a disintegrant which is croscarmellose sodium;
- at least one filler selected from microcrystalline cellulose, silicified microcrystalline cellulose and mixture thereof;
- a lubricant which is magnesium stearate; and
- a glidant which is colloidal anhydrous silica.

8. The solid dosage form according to any one of the preceding claims, which contains:
- 20-40 % (w/w), preferably 30-40 % (w/w), of solid dispersion of apalutamide comprising or consisting of apalutamide and hydroxypropyl methylcellulose phthalate, preferably in the weight ratio about 1:3;
- 3-15 % (w/w), preferably 3-7 % (w/w), of disintegrant which is croscarmellose sodium;
- 20-70 % (w/w), preferably 55-60 % (w/w), of filler selected from microcrystalline cellulose, silicified microcrystalline cellulose and mixture thereof;
- 0.2-7 % (w/w), preferably 0.2-1 % (w/w), of lubricant which is magnesium stearate; and
- 0.2-7 % (w/w), preferably 0.5-1.5 % (w/w), of glidant which is colloidal anhydrous silica.

9. A method for preparing the solid dosage form of apalutamide, said method comprising the following steps:
a) providing a solution of apalutamide and HPMCP in a solvent,
b) isolating a solid dispersion of apalutamide and HPMCP,
c) combining the solid dispersion with at least one pharmaceutically excipient,
d) producing the solid dosage form.

10. The method according to claim 9, wherein the solvent in step a) is a mixture of methanol and dichloromethane, more preferably a mixture of methanol and dichloromethane 6:4 (w:w).

11. The method according to claim 9 or 10, wherein the method of isolating the solid dispersion in step b) is a spray-drying process, comprising the following steps:
b1) injecting the solution of apalutamide and HPMCP obtained in step a) to a spray dryer,
b2) atomization of the solution to create droplets,
b3) drying the droplets in a heated air stream,
b4) collecting the dried particles.

12. The method according to any one of claims 9 to 11, wherein in step c), the solid dispersion is first blended with a part (50 to 80 %) of the glidant, this blend is further blended with a part (25 to 75 %) of the disintegrant and with the a part (50 to 80 %) of the filler, the resulting mixture is subjected to dry granulation, and the dry granulate is then blended with the remaining amounts of the filler, glidant and disintegrant, and finally the mixture is blended with the lubricant.

13. The solid dosage form according to any one of claims 1 to 8 for use in the treatment of prostate cancer, preferably in the treatment of metastatic castration-resistant prostate cancer.
